Europäisches Patentamt

European Patent Office

Office européen des brevets

(19)

(11) Veröffentlichungsnummer: **0 272 434**
**A2**

(12) **EUROPÄISCHE PATENTANMELDUNG**

(21) Anmeldenummer: **87116685.6**

(22) Anmeldetag: **12.11.87**

(51) Int. Cl.4: **A61B 17/16**

(30) Priorität: **20.11.86 DE 3639696**

(43) Veröffentlichungstag der Anmeldung:
**29.06.88 Patentblatt 88/26**

(84) Benannte Vertragsstaaten:
**AT BE CH DE ES FR GB GR IT LI LU NL SE**

(71) Anmelder: **List, Heinz-Jürgen**
**Lindenstrasse 56**
**D-7990 Friedrichshafen 1(DE)**

(72) Erfinder: **Richter, Ulrich**
**Ludolfingerstrasse 14**
**D-8500 Nürnberg 30(DE)**

(74) Vertreter: **Göbel, Matthias, Dipl.-Ing.**
**Pruppacher Hauptstrasse 5-7**
**D-8501 Pyrbaum-Pruppach(DE)**

(54) **Knochenbohrmaschine.**

(57) Bei einer Knochenbohrmaschine mit einer im Bohrmaschinengehäuse durch einen Elektromotor drehbaren Abtriebswelle, die ein Spannelement für ein Bohrwerkzeug antreibt, ist zur Schaffung von Maßnahmen zu einer stoßfreien und umschaltbaren Abtriebswelle vorgesehen, daß die Motorwelle (3) durch ein flexibles Kraftübertragungsglied (5) mit der Abtriebswelle (6) in Verbindung steht und daß die Abtriebswelle (6) über mindestens ein umschaltbares Planetenradgetriebe einen das Spannelement aufnehmenden Wellenstumpf (24) dreht.

Fig. 1

EP 0 272 434 A2

## Knochenbohrmaschine

Die Erfindung betrifft eine Knochenbohrmaschine mit einer im Bohrmaschinengehäuse durch einen Elektromotor drehbaren Abtriebswelle, die ein Spannelement für ein Bohrwerkzeug antreibt.

Bei einer bekannten chirurgischen Handbohrmaschine (DE-OS 3 317 398) steht der Antriebsmotor über ein Kegelräderpaar permanent mit einer ein Spannfutter aufweisenden Abtriebswelle in Verbindung. Drehzahländerungen der Abtriebswelle erfolgen bei dieser Knochenbohrmaschine durch Veränderung des Widerstandswertes eines den Stromkreis des Antriebsmotors beeinflußenden Potentiometers. Diese über den gesamten Drehzahlbereich sich erstreckende Regelung der Motordrehzahl führt jedoch zu ungünstigen Drehmomentbeeinflussungen. Weiter können sich stoßartige Drehmomentänderungen über die Kegelräder direkt auf die Abtriebswelle und das Spannfutter übertragen, was die auf chirurgischem Gebiet empfindlichen Bohrvorgänge negativ belastet.

Es ist Aufgabe der Erfindung, bei Knochenbohrmaschinen die Abtriebswelle stoßfrei und umschaltbar auszubilden und beliebig Bohrwerkzeuge und längenorientiertes Material zu fassen.

Der Erfindung gemäß ist diese Aufgabe dadurch gelöst, daß die Motorwelle durch ein flexibles Kraftübertragungsglied mit der Abtriebswelle in Verbindung steht und daß die Ab triebswelle über mindestens ein umschaltbares Planetenradgetriebe einen das Spannelement aufnehmenden Wellenstumpf dreht. Nach bevorzugter Ausführung sind zwischen der Abtriebswelle und dem Wellenstumpf zwei Planetenradgetriebe hintereinander angeordnet, die ein gemeinsames Sonnenrad und im axialen Abstand voneinander zwei unabhängige drehbewegliche Trägerscheiben für die Planetenräder der beiden Planetenradgetriebe aufweisen und die Planetenräder des ersten Planetenradgetriebes mit einer auf der Abtriebswelle ausgebildeten Zahnung und die Planetenräder des zweiten Planetenradgetriebes mit einer an der Trägerscheibe für die Planetenräder des ersten Planetenradgetriebes ausgebildeten Zahnung in Eingriff stehen, während die Trägerscheibe der Planetenräder des zweiten Planetenradgetriebes mit dem Wellenstumpf drehfest verbunden ist. Auf diese Weise ist erreicht, daß störende Stoßbelastungen zwischen Antriebsmotor und Abtriebswelle vermieden sind und durch die mechanischen Umschaltungen Drehzahländerungen unter Beibehaltung der Motordrehzahl und des Drehmoments vornehmbar sind.

Dabei führt das flexible Kraftübertragungsglied, z. B. ein Zahnriemen, zu einer ersten Getriebestufe, die spielfrei ist und zu einem weichen Anlaufen des Spannelements beiträgt. Außerdem sind Toleranzprobleme hinsichtlich des Abstandes von Motorwelle und Abtriebswelle bzw. der Achsenlagen beseitigt und eine Getriebestufe mit geringen Laufgeräuschen geschaffen. Als flexibles Kraftübertragungsglied kann z. B. ein Riementrieb Anwendung finden, der zweckmäßig über Ritzel der Motorwelle und Antriebswelle mit profilierten Umfangsflächen geführt ist. Auch kann das Kraftübertragungsglied selbst ritzelseitig Profilierungen tragen. Es versteht sich, daß elektrische Drehzahlregelungen für den Antriebsmotor beibehaltbar sind, die nunmehr durch Drehzahlveränderungen vermittels Umschaltungen der Planetenradgetriebe überlagerbar sind.

In Ausgestaltung der Bohrmaschine ist vorgesehen, das Sonnenrad durch eine schaltbare Kuppelscheibe mit der Abtriebswelle kraft-, form- oder reibschlüssig zu verbinden. Zweckmäßig ist die Kuppelscheibe zwischen zwei Stellungen axial frei verschieblich und frei drehbar auf der Abtriebswelle angeordnet und auf mit dem Sonnenrad fest verbundenen Führungsstiften gegenüber dem Sonnenrad unverdrehbar gelagert und die Kuppelscheibe in der einen Stellung an einer mit der Abtriebswelle drehfest verbundenen Nockenscheibe anlegbar und in der anderen Stellung an gehäusefeste Nocken fixierbar. Zu Umschaltungen bedarf es somit lediglich einer einfachen Verschiebebewegung der Kuppelscheibe. Diese axialen Verschiebebewegungen lassen sich, z. B. mittels eines Exzentertriebes erreichen, der bevorzugt durch ein aus dem Bohrmaschinengehäuse herausragendes Betätigungsglied manuell drehbar ist. Die Betätigung der Kuppelscheibe zu Umschaltungen kann auch anderweitig, z. B. über ein Hebelgetriebe erfolgen.

In Ausbildung der Knochenbohrmaschine ist vorgesehen, die Abtriebswelle und den Wellenstumpf als Hohlwellen auszubilden und in der Abtriebswelle ein mit dem Wellenstumpf verbundenes Führungsrohr anzuordnen. Auf diese Weise erhält die Abtriebswelle ein relativ großes Biegemoment und über die Mittelöffnung des Führungsrohres ist die Durch leitung von längenorientiertem Material, wie Bohrdrähten, Lichtleitfasern für Endoskope störungsfrei und schonend möglich.

Es entspricht der Erfindung, die Bohrdrähte entweder manuell vorzuschieben oder maschinell, insbesondere von der Drehrichtung abhängig selbsttätig vorzuschieben oder zurückzuholen. Selbsttätige Verschiebungen der Bohrdrähte sind durch ein motorisch drehbares Klemmrollengesperre bewirkbar.

Die Erfindung ist anhand eines Ausführungsbeispiels in der Zeichnung verdeutlicht. Es zeigen:

Fig. 1 einen Teilschnitt einer Knochenbohrmaschine und

Fig. 2 eine erste Getriebestufe, perspektivisch.

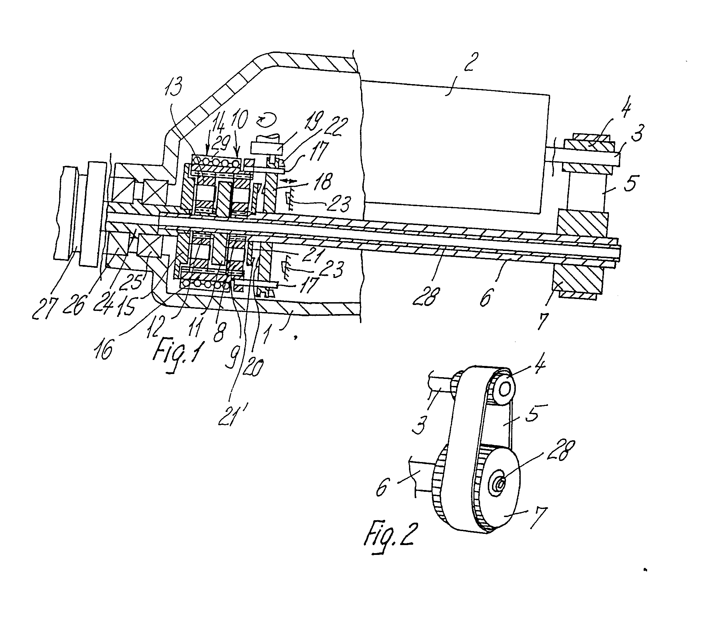

In Fig. 1 ist mit 1 ein Knochenbohrmaschinengehäuse bezeichnet, das durch zusammengefügte Gehäusehälften gebildet ist. Das Gehäuse 1 nimmt einen Elektromotor 2 auf, der durch elektrische Schaltelemente (nicht gezeigt) an eine Stromquelle (nicht gezeigt) anlegbar bzw. in Drehzahlen regelbar ist. Die Welle 3 des Elektromotors 2 trägt ein Ritzel 4, das über einen Riemen 5 mit einem auf einer im Maschinengehäuse 1 gelagerten Abtriebswelle 6 fest angeordneten Getrieberad 7 in Verbindung steht. Die Abtriebswelle 6 ist achsparallel zur Welle 3 ausgebildet und beim Ausführungsbeispiel als Hohlwelle ausgeführt. An ihrem dem Getrieberad 7 abgewandten Ende trägt die Abtriebswelle 6 eine Zahnung 8, die mit Plahetenrädern 9 eines ersten Planetenradgetriebes 10 kämmen. Die Planetenräder 9 sind an einer frei drehbaren Trägerscheibe 11 angeordnet. Die Trägerscheibe 11 kämmt mit einer Zahnung 12 mit weiteren Pla netenrädern 13 eines zweiten Planetenradgetriebes 14, die durch eine Trägerscheibe 15 getragen sind. Planetenräder 9, 13 und Trägerscheiben 11, 15 sind durch ein gemeinsames Sonnenrad 16 umfaßt, das mit den Planetenrädern 9, 13 der beiden Planetenradgetriebe 10 und 14 gleichzeitig in Eingriff steht. Das Sonnenrad 16 steht über Führungsstifte 17 mit einer Kuppelscheibe 18 in Verbindung, die zur Abtriebswelle frei drehbar und auf dieser frei verschiebbar ist. Die Kuppelscheibe 18 ist durch einen Exzentertrieb 19 auf der Abtriebswelle 6 und auf den Führungsstiften 17 zwischen zwei Kuppelstellungen verschieblich und vermittels Nocken 20 in der einen Kuppelstellung mit einer auf der Abtriebswelle 6 fest angeordneten Nockenscheibe 21 kuppelbar und in der anderen Kuppelstellung über Nocken 22 mit maschinengehäusefesten Nocken 23 in Eingriff bringbar.

Mit der Trägerscheibe 15 steht ein Wellenstumpf in Verbindung, der im Maschinengehäuse 1 durch Kugellager 25 od. dgl. gestützt und vermittels einer Dichtung 26 dicht geführt ist. Der Wellenstumpf 24 dient als Träger eines Spannfutters 27 für die Fixierung eines Bohrwerkzeugs. Die Abtriebswelle 6 nimmt in der Mittelöffnung ein Führungsrohr 28 auf, das sich bis zum Wellenstumpf 24 erstreckt. Das Führungsrohr 28 ermöglicht die Aufnahme und Durchführung von längenorientierten Materialien durch die Abtriebswelle 6 zum Spannfutter 27 bzw. Spannzange der Spannkonus.

In der in Fig. 1 gezeigten Stellung dreht der Elektromotor 2 über das Ritzel 4, den Riemen 5 und das Getrieberad 7 die Abtriebswelle 6. Ist die Kuppelscheibe 18 durch Be tätigen des Exzentertriebs 19 mit ihren Nocken 20 an die Nocken 21' der Nockenscheibe 21 angelegt, so nimmt bei den Umläufen der Abtriebswelle 6 die Nockenscheibe 21 die Kuppelscheibe 18 mit und durch diese wird über die Führungsstifte 17 das Sonnenrad 16 gedreht. Durch das Sonnenrad 16 werden die Planetenräder 9, 13 und die Trägerscheiben 11, 15 und weiter der Wellenstumpf im Verhältnis 1:1 zur Abtriebswelle mitgenommen.

Durch Verschieben der Kuppelscheibe 18 mittels des Exzentertriebs 19 bis zur Anlage der Nocken 22 an die gehäusefesten Nocken 23 ist die Kuppelscheibe 18 fixiert und über die Führungsstifte das Sonnenrad 16 unverdrehbar gehalten. Über die Zahnung 8, die Planetenräder 9 und Trägerscheibe 11, der Zahnung 12, den Planetenrädern 13 und der Trägerscheibe 15 wird auf den Wellenstumpf 24 ein Drehmoment mit Drehzahluntersetzung ausgeübt. Es versteht sich, daß die beiden Planetenradgetriebe 10, 14 aus einem beliebigen Werkstoff, z. B. einem metallischen Werkstoff oder einem Kunststoff gebildet sein können. Durch entsprechende Bemessung der Planetenradgetriebe sind vorbestimmte Untersetzungen erzielbar.

Die Knochenbohrmaschine weist somit eine erste flexible Getriebestufe und eine mechanisch wirkende zweite Getriebestufe auf, wobei die erste Getriebestufe zu einem weichen und schlupffreien Anlaufen ohne Toleranzprobleme führt. Mit 29 ist ein maschinengehäusefestes Rollenlager für das Sonnenrad 16 bezeichnet.

## Ansprüche

1. Knochenbohrmaschine mit einer im Bohrmaschinengehäuse durch einen Elektromotor drehbaren Abtriebswelle, die ein Spannelement für ein Bohrwerkzeug antreibt, dadurch gekennzeichnet, daß die Motorwelle (3) durch ein flexibles Kraftübertragungsglied (5) mit der Abtriebswelle (6) in Verbindung steht und daß die Abtriebswelle (6) über mindestens ein umschaltbares Planetenradgetriebe einen das Spannelement aufnehmenden Wellenstumpf (24) dreht.

2. Knochenbohrmaschine nach Anspruch 1, dadurch gekennzeichnet, daß zwischen der Abtriebswelle (6) und dem Wellenstumpf (24) zwei Planetenradgetriebe (10, 14) hintereinander angeordnet sind, die ein gemeinsames Sonnenrad (16) und im axialen Abstand zwei unabhängige drehbewegliche Trägerscheiben (11, 15) für die Planetenräder (9, 13) der beiden Planetenradgetriebe (10, 14) aufweisen und daß die Planetenräder (9) des ersten Planetenrad getriebes (10) mit einer auf der Abtriebswelle (6) ausgebildeten Zahnung (8) und die Planetenräder (13) des zweiten Planetenradgetriebes (14) mit einer an der Trägerscheibe (15) für die Planetenräder (9 ) des ersten Planetenradgetriebes (10) ausgebildeten Zahnung (12) kämmen und daß die Trägerscheibe (15) der Planetenräder (13) des zweiten Planetenradgetriebes (14) mit dem Wellenstumpf (24) drehfest verbunden ist.

3. Knochenbohrmaschine nach Anspruch 2, dadurch gekennzeichnet, daß das Sonnenrad (16) vermittels einer schaltbaren Kuppelscheibe (18) mit der Abtriebswelle (6) kraft-, form-oder reibschlüssig verbindbar ist.

4. Knochenbohrmaschine nach Anspruch 2 und 3, dadurch gekennzeichnet, daß die schaltbare Kuppelscheibe (18) zwischen zwei Stellungen axial frei verschieblich und frei drehbar auf der Abtriebswelle (6) angeordnet und auf mit dem Sonnenrad (16) fest verbundenen Führungsstiften (17) 'gegenüber dem Sonnenrad (16) unverdrehbar gelagert ist und daß die Kuppelscheibe (18) in der einen Stellung an eine mit der Abtriebswelle (6) drehfest verbundenen Nockenscheibe (21) anlegbar und in der anderen Stellung an gehäusefeste Nocken (23) fixierbar ist.

5. Knochenbohrmaschine nach Anspruch 2, 3 und 4, dadurch gekennzeichnet, daß die Kuppelscheibe (18) mittels eines Exzentertriebes (19) auf der Abtriebswelle (6) und den Führungsstiften (17) verschiebbar ist und daß der Exzentertrieb (19) durch ein aus dem Bohrmaschinengehäuse (1) herausragendes Betätigungsglied drehbar ist.

6. Knochenbohrmaschine nach Anspruch 1, dadurch gekennzeichnet, daß die Motorwelle (2) durch einen Riemen (5) aus einem flexiblen Werkstoff mit der Abtriebswelle (6) verbunden ist.

7. Knochenbohrmaschine nach Anspruch 6, dadurch gekennzeichnet, daß der Riemen (6) auf auf der Motorwelle (2) und der Abtriebswelle (6) angeordnete Ritzel (4, 7) mit profilierten Umfangsflächen geführt ist.

8. Knochenbohrmaschine nach Anspruch 1, dadurch gekennzeichnet, daß die Abtriebswelle (6) und der Wellenstumpf (24) als Hohlwellen ausgebildet sind und daß in der Abtriebswelle (6) ein mit dem Wellenstumpf (24) verbundenes Führungsrohr (28) angeordnet ist.

9. Knochenbohrmaschine nach Anspruch 1, dadurch gekennzeichnet, daß der Wellenstumpf (24) durch Kugel-oder Rollenlager (25) im Bohrmaschinengehäuse (1) gestützt und mittels eines Dichtringes (26) dicht geführt ist.

Fig. 1

Fig. 2